# EUROPEAN PATENT APPLICATION

(11) **EP 3 075 446 A1**
(43) Date of publication of application: **05.10.2016**
(21) Application number: 15161702.4
(22) Date of filing: 30.03.2015
(51) Int. Cl.: B01J 10/00, B01J 19/00, B01J 19/24, C07C 273/04

(54) **UREA SYNTHESIS REACTOR AND METHOD OF REVAMPING**

(71) Applicant: Casale SA, 6900 Lugano (CH)
(72) Inventor: Rizzi, Enrico, 22070 Casnate con Bernate (CO) (IT); Scotto, Andrea, 6932 Breganzona (CH)
(74) Representative: Zardi, Marco

(57) **Abstract**

Reactor for the synthesis of urea from ammonia and carbon dioxide, comprising a plurality of perforated baffles (4) which divide the inside of the reactor into compartments, wherein the baffles form part of a structurally independent cartridge (3); a corresponding revamping method consists in preparing the cartridge comprising the baffles and inserting it inside the reactor, through the full-opening flange.

## Description

### Field of application

The invention relates to the field of reactors for the synthesis of urea from ammonia and carbon dioxide.

### Prior art

The processes and apparatus for the synthesis of urea from ammonia (NH₃) and carbon dioxide (CO₂) are described in the literature, for example in Ulmann's Encyclopedia of Industrial Chemistry, Wiley-VCH Verlag, vol. A27.

A known and widely used configuration for the urea reactor comprises a series of perforated baffles which are substantially perpendicular to the axis of the reactor and divide up the inside of the reactor into a certain number of compartments, typically from five to twenty and more frequently from eight to fifteen compartments, depending on the dimensions of the reactor. Said baffles are also termed "plates".

The presence of the baffles and the consequent partition of the reactor into compartments give rise to a series of positive effects. These effects consist essentially in a beneficial redistribution of the liquid and gaseous phases inside the reactor, thus improving the heat and mass exchanges between the two phases. The baffles may have simple or more sophisticated forms, for example a particularly advantageous baffles configuration was devised by the applicant and described in EP 0 495 418.

The construction of the baffles poses a number of technological problems also because they are prone to wear and corrosion and it is desirable they may be replaceable with relative ease. A known and widely used solution consists in providing an anchoring ring for each baffle; the baffle is bolted to the respective ring, and the ring is in turn bolted to support brackets which are welded to the anti-corrosion internal lining of the reactor.

This constructional design is not per se problematic for new reactors, which may be designed with the necessary support brackets for the rings. In the case of revamping of an existing reactor, however, a number of obstacles are encountered. The existing supports inside the reactor, in fact, cannot be generally used, either because they are damaged (for example corroded) and should be replaced, or because revamping envisages an arrangement of the plates which is different from the original arrangement. In this case it would be necessary to weld new supports to the reactor lining and this would constitute a difficult and delicate operation.

In fact, it involves performing welds inside the reactor, which is a per se complex operation and in particular which poses problems with regard to the quality of the welds since it directly affects the protective lining of the shell. It is known that welds constitute potential weak points with regard to the resistance to corrosion and therefore an imperfect weld performed on the lining could give rise to an initial corrosion point with serious consequences; if the corrosion develops to the extent of affecting the pressurized shell and is not identified promptly, the consequences may be catastrophic, with risks for safety and a prolonged plant shut-down with serious economic loss.

In order to avoid these risks, the welds must be performed to the prescribed standard by specialized personnel, but this never provides any absolute guarantee as to the absence of corrosion and greatly increases the working time and costs of revamping. By way of example, the replacement of a set of perforated plates of a urea reactor for a large plant may require 10-15 days.

In some cases, especially in partial-opening reactors, the plates are formed in segments so as they may be introduced through the manhole. This further increases the working time. In general, in the case of a partial-opening urea reactor in which the new plates must be installed, the first thing to check is that the supports or brackets are at proper height and have the correct orientation. Once this condition has been checked, it is necessary to introduce the support ring for each plate, which in a partial-opening reactor is formed in segments and therefore must be welded inside the reactor. After welding or bolting the support ring to the brackets, the plates may be mounted. Each plate consists of segments bolted together and bolted onto the support ring.

The aforementioned baffles, which are perforated with given patterns, pose another problem. It is known that anomalous operating conditions of the reactor may generate sudden pressure waves which are propagated in the fluid, resulting in the transmission of considerable forces to the baffles. These forces may deform or damage the baffles, and expose the reactor to an even more serious risk which is represented by the damaging of the lining. In fact the baffles are anchored to the lining by means of the support brackets. The brackets are welded directly to the lining and this means that the collapse of a bracket could adversely affect the lining and create a zone which is vulnerable to corrosion, with serious risk of collapse of the pressurized shell.

In order to avoid this risk, at present controlled-breakage connections are provided between the baffles and the respective support ring. Said connections are designed to break in the presence of pressure waves with a force greater than a given threshold, regarded as dangerous for the integrity of the lining, thus causing separation of the baffles. This solution, however, has the drawback of being costly and, moreover, does not provide a total guarantee that the lining is preserved, since the stress conditions are difficult to predict. A certain margin of safety must be adopted when determining the collapse threshold and this means that the threshold must be relatively low and that, in the event of anomalous operation or triggering of a pressure wave, the separation of one or more baffles is a relatively probable event. It is clear that the separation of a baffle is not per se a catastrophic event, like the collapse of a pressurized shell, but in any case it is a serious damage which sooner or later must be repaired, resulting in shut-down of the reactor and major costs.

### Summary of the invention

The object of the invention is to overcome these problems. A first object of the invention is to facilitate the construction of urea reactors and maintenance thereof, as well as the revamping of the existing reactors, in particular with regard to the replacement of the internal baffles. Another object is to reduce the serious risks associated with anomalous operation and unexpected pressure waves.

The idea underlying the invention is to provide a structurally independent cartridge which can be inserted inside a full-opening urea reactor.

The objects of the invention are therefore achieved with a vertical reactor for the synthesis of urea from ammonia and carbon dioxide, comprising a plurality of perforated baffles which divide the inside of the reactor into compartments, characterized in that it comprises a structurally independent cartridge which is inserted inside the reactor and which supports said baffles.

The cartridge advantageously comprises a support structure and the perforated baffles are firmly fixed to said support structure. In a preferred embodiment the structure comprises a plurality of axial bars arranged in the form of a cage around the baffles. Preferably said bars are fixed to the peripheral edge of the rings which support the baffles.

In an even more preferred embodiment, the structure is divided into at least two longitudinal (axial) sections. A structure divided into sections has the advantage of simplifying transport and insertion inside the reactor without using large-size cranes. It should be noted that the reactor is vertical and has a considerable height, on average of around 50-60 m; this means that the assembly (insertion inside the reactor) of a single-piece structure requires a lifting equipment able to reach a height of more than 120 m. A crane with such large dimensions is usually unavailable inside the plant; hiring such equipment is very costly and must be planned a long time in advance.

Another aspect of the invention also relates to a method for revamping a full-opening urea reactor. The method comprises: providing a structurally independent cartridge comprising a plurality of perforated baffles and a structure for supporting said baffles: inserting said cartridge inside the reactor shell through said full opening. Preferably the cartridge comprises a plurality of longitudinal portions which may be assembled so as to form the entire cartridge before insertion inside the reactor or which may be inserted one at a time and joined together inside said reactor.

The invention eliminates the long and difficult operations of welding the support brackets inside the reactor. The invention is particularly advantageous for revamping existing urea reactors.

At the construction site, the cartridge is assembled outside the reactor and the plant is stopped in order to open the reactor and replace the plates only once the structure has been assembled externally. Generally the structure and the plates are disassembled and packaged for delivery. It is possible to perform a pre-assembly in the factory in order to check that there are no design or construction defects and to reduce the risk of problems during the work on-site (at the plant).

The main advantage of the invention is that the replacement of a set of perforated baffles inside a reactor becomes possible within a time span of 2-3 days compared to 10-15 days of the prior art. The downtime of the reactor is kept to a minimum because the cartridge may be prepared while the reactor is in function, and basically the reactor must be stopped only for the possible preparation of the support structure and the insertion of the cartridge.

It should be noted, however, that the invention is applicable also to the construction of new reactors, wherein the provision of a structurally independent cartridge, which can be inserted inside the reactor shell, simplifies the construction and reduces the costs. The invention is attractive also for a new reactor because it is clear that the maintenance of the reactor will be simpler since the internal baffles may be replaced much more easily.

The invention is applicable to all vertical-axis urea reactors and is applicable to the known various types of urea plants, including self-stripping plants (Snamprogetti), CO₂-stripping plants and variants thereof.

Advantageously, the cartridge according to the invention, when inserted inside the urea reactor, rests on a support specifically provided on the bottom of the reactor. Preferably, said support has the form of a continuous ring, even though other constructional designs are possible, for example using a series of brackets projecting from the shell. In the case of new reactors, said support is provided during the design stage; in the case of revamping of an existing reactor, if a suitable support is not available inside the reactor, said support is formed in loco; the difficulty associated with forming the support inside the reactor is however less than that of the processes of the known art which involve welding the various brackets at different heights along the entire axis of the reactor.

One of the preferred applications of the invention consists in replacing perforated plates of the conventional type with improved baffles, for example made in accordance with EP 495 418.

The cartridge according to the invention, when inserted inside the reactor, is mechanically detached from the internal lining of said reactor. The term "detached" denotes that the cartridge has no points directly fixed to the reactor lining, unlike the conventional brackets. Therefore the cartridge does not transmit stress to the lining. The constraint existing between the cartridge and the reactor shell is substantially represented only by the cartridge bearing on the bottom ring or support. This constitutes another major advantage because, in the event of an anomalous pressure wave, the thrusts acting on the plates are not transmitted to the reactor lining. The invention therefore substantially reduces the risk of damage to the anti-corrosion lining which represents a vital part of the reactor.

The advantages will emerge even more clearly with the aid of the detailed description below relating to a number of preferred embodiments.

### Description of the figures

Fig. 1 is a diagram of a urea reactor according to a preferred embodiment of the invention containing a cartridge which supports a series of perforated plates.
Fig. 2 shows the cartridge extracted from the reactor according to Fig. 1.
Fig. 3 shows an example of a procedure for revamping a urea reactor according to the invention.

### Detailed description

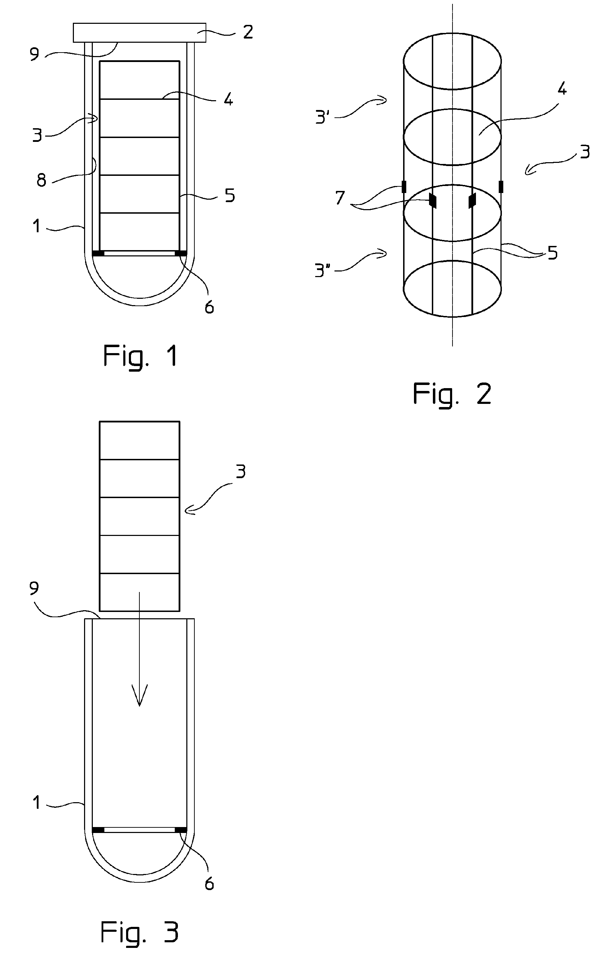

Fig. 1 shows in schematic form a vertical reactor for the synthesis of urea from ammonia and carbon dioxide, comprising a shell 1 with a flange 2 having a full opening 9. The terms "full opening" denotes an opening having approximately the same diameter as the shell 1.

The reactor contains a cartridge 3 which comprises a plurality of perforated plates 4 and a respective support cage formed by bars 5. The cartridge 3 rests on a support ring 6 at the base of the reactor.

The perforated plates 4 divide the internal volume of the reactor into compartments. The plates 4 may be made with forms known per se or more generally as baffles which are permeable to the liquid and gas contained in the reactor.

The cartridge 3 (Figs. 1-2) is a stand-alone component which is structurally independent of the reactor. It should be noted that the cartridge 3 has no points directly fixed to the internal lining 8 of the shell 1. The weight of the cartridge is supported by the support ring 6, but there are no elements which are firmly fastened to the lining 8. This results in a dual advantage because no welds involving the lining 8 are performed and, in the event of anomalous stresses acting on the plates 4, the resultant forces are not transmitted to said lining.

In a preferred embodiment (Fig. 2) the cartridge 3 comprises a plurality of longitudinal sections, for example two sections 3' and 3", which are joined together by junctions 7. This expedient reduces the dimensions of the single sections which can be inserted more easily inside the reactor.

Fig. 3 shows in schematic form a possible revamping of an existing reactor. The full-opening flange 2 is open and the cartridge 3, if necessary after joining together of the sections 3' and 3", is inserted inside the shell 1. Before the insertion of the cartridge 3, the support ring 6 is arranged inside the reactor.

The described procedure of revamping may be used to replace existing baffles (for example when damaged or corroded) or to add baffles to a reactor originally unprovided.

The reactor may form part of a high-pressure synthesis loop of a urea plant, for example a plant of the ammonia stripping or self-stripping type (also referred to as Snamprogetti plant) or a CO₂-stripping plant.

## Claims

1. Vertical reactor for the synthesis of urea from ammonia and carbon dioxide, comprising a plurality of perforated baffles (4) which divide the inside of the reactor into compartments, **characterized in that** it comprises a structurally independent cartridge (3) which is inserted inside the reactor and which supports said baffles.

2. Reactor according to claim 1, wherein said cartridge comprises a support structure and the perforated baffles are firmly fixed to said structure.

3. Reactor according to claim 2, wherein said structure comprises a plurality of axial bars (5) arranged in the form of a cage around the perforated baffles, said bars being fixed to the peripheral edge of the baffles.

4. Reactor according to anyone of claims 2 and 3, wherein said cartridge comprises at least two longitudinal portions (3', 3").

5. Reactor according to anyone of claims 1-4, **characterized in that** the cartridge (3) is mechanically detached from the shell (1) of said reactor.

6. Reactor according to claim 5, wherein the cartridge has no points directly fixed to the internal lining of said shell (1).

7. Reactor according to claim 6, **characterized in that** the cartridge (3) rests on a support structure (6) located on the bottom of the reactor.

8. Method for revamping a urea reactor comprising a shell (1) with a full opening (9), comprising:
- arranging a structurally independent cartridge (3) comprising a plurality of perforated baffles (4) and a support structure (5) for said baffles.
- inserting said cartridge inside the reactor shell through said full opening.

9. Method according to claim 8, wherein the cartridge comprises a plurality of longitudinal portions (3', 3") which are assembled so as to form the entire cartridge before insertion inside the reactor or which are inserted one at a time and joined together inside the reactor.

10. Method according to claim 8 or 9, also comprising the provision of a support structure (6) for the cartridge (3) inside the reactor, before the insertion of the cartridge.
